(19) Europäisches Patentamt
European Patent Office
Office européen des brevets

(11) EP 4 726 125 A1

(12) **EUROPEAN PATENT APPLICATION**

(43) Date of publication:
15.04.2026 Bulletin 2026/16

(21) Application number: 25207400.0

(22) Date of filing: 08.10.2025

(51) International Patent Classification (IPC):
E03F 7/00 (2006.01)    F17D 5/02 (2006.01)
G01M 3/28 (2006.01)    G01N 33/18 (2006.01)
G05B 13/04 (2006.01)    G06F 30/27 (2020.01)
G06N 20/00 (2019.01)    G06F 21/57 (2013.01)

(52) Cooperative Patent Classification (CPC):
F17D 5/02; E03F 7/00; G01M 3/2815; G01N 33/18;
G06F 30/27; G06N 7/01; G06N 20/00; G06F 21/577

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 09.10.2024 IT 202400022473

(71) Applicant: Idea-Re S.r.l.
00198 Roma (IT)

(72) Inventors:
• GARINEI, Alberto
  Roma (IT)
• MARINI, Andrea
  Roma (IT)
• SPEZIALI, Stefano
  Roma (IT)
• MARCONI, Marcello
  Roma (IT)
• PICCIONI, Emanuele
  Roma (IT)

(74) Representative: Caforio, Giuseppe
Via Bartolo, 10
06122 Perugia (PG) (IT)

(54) **METHOD FOR MONITORING PRESSURIZED AND/OR GRAVITY-FED NETWORK INFRASTRUCTURES**

(57) A method for monitoring epidemiologically and/or toxicologically and/or pollutants in network infrastructures, either under pressure or under gravity, such as sewers. The method is based on the continuous analysis of a set of network nodes and is accompanied by additional random analyses of other nodes in case the continuous analysis would reveal anomalies. These other nodes are chosen as the most significant for identifying the sources of the anomaly, and are chosen in compliance with the constraints imposed by sampling and analysis logistics. This monitoring method can be run not only on traditional computers, but also on quantum computers with very low execution time, and provides not only the source nodes of a potential infection/pollution, but also a probability for each node of being involved in the potential infection/pollution.

Figure 1

Processed by Luminess, 75001 PARIS (FR)

EP 4 726 125 A1

## Description

## Scope of the Invention

The proposed method applies to the technical sector of pressure and/or gravity networks, particularly sewer systems.

## State of the Art

Current theoretical models for epidemiological and/or toxicological and/or pollutant monitoring of sewer systems are based on semi-permanent sensors installed on the network. Sensor data are generally read remotely, and these theoretical models identify any anomalies in the network based on the sensor values. If an anomaly is identified, or if standard monitoring protocols are implemented, laboratory analyses are performed on wastewater samples taken from the network at specific nodes that, according to the theoretical network model, are the most significant to analyze. Therefore, one or more work teams must physically travel to these specific nodes, collect samples, and transport them to the laboratory; this is commonly referred to as "mobile sampling".

Current theoretical models for epidemiological and/or toxicological and/or pollutant monitoring of sewer networks are logistics-independent. Therefore, the model may require mobile sampling of nodes that could be currently inaccessible (for example, due to maintenance work on the access road to the node), or the model may require mobile sampling of nodes that cannot be reached in a single work day (for example, too far apart or in heavy traffic conditions), or the like.

A primary aim of the present invention is to provide a theoretical model for epidemiological and/or toxicological and/or pollutant monitoring of sewer networks that incorporates mobile sampling logistics.

Current theoretical models for epidemiological and/or toxicological and/or pollutant monitoring of sewer networks take a long time to provide output. Therefore, in the event of variable network conditions or logistical impossibility of reaching a network node, the monitoring will be incomplete or of limited use.

A second purpose of the present invention is to provide a theoretical model for epidemiological and/or toxicological and/or pollutant monitoring of sewer networks that is significantly faster than current models.

Current theoretical models for epidemiological monitoring of sewer networks are aimed at identifying the source node of any anomaly detected in the network, but they do not provide a complete mapping of the epidemiological situation or its evolution over time.

A third purpose of the present invention is to provide a complete mapping of the epidemiological and/or toxicological situation and/or the presence of pollutants.

Having detected an anomaly at some node, one may be interested in verifying how the anomaly evolves over time, both at the level of individual anomalous nodes and across the entire network. A fourth aim of the present invention is to provide the evolution of the epidemiological and/or toxicological situation and/or the presence of pollutants over time.

## Summary of the Invention

These purposes and other advantageous aspects are achieved in the present invention according to the technical solution disclosed and claimed herein.

A method is described where the choice of mobile sampling nodes and logistics are simultaneously optimized.

The method is based on a mathematical function, hereinafter referred to as network energy, to be optimized, while respecting the constraints defined by the availability of resources for performing the sampling/-measurements. The solution that optimizes said network energy function is the set of nodes at which said mobile sampling has to be performed (second purpose of the invention).

This network energy is defined as a quadratic function, therefore the search for its maximum/minimum points can be performed using combinatorial optimization algorithms already known in the scientific literature for QUBO (Quadratic Unconstrained Optimization Problem)-type problems.

The optimization algorithm's formulation as a "Quadratic Unconstrained Optimization Problem" (QUBO) allows it to be processed not only on classical computers but also on quantum computers. For example, the optimization problem can be solved using Quantum Annealers, computational devices designed to solve combinatorial optimization problems, through a process that uses quantum fluctuations to find the global minimum of a given objective function. Quantum Annealing starts from a quantum mechanical superposition of all possible states and evolves over time following the natural evolution of the physical system.

Therefore, the optimization algorithm runs quickly, and if sampling at one or more nodes indicated by the algorithm is not possible due to unforeseeable critical issues encountered in field operations, the optimization algorithm can be re-executed, excluding the possibility of sampling at the nodes where such critical issues are encountered (second purpose of the invention).

A sewer system is a network that transports wastewater from all possible sources to a water treatment plant. The endpoint of the graph, reachable from every node in the network, corresponds to the network's treatment plant. Each source is associated with a node in the network. All nodes correspond to manholes, are physically accessible to humans, and are connected by sewer pipes, generally under city streets. This network can be modeled as a directed acyclic graph. In general, the network does not need to have the structure of a tree, as the underlying graph can have cycles.

If the presence of an unwanted chemical or biological substance is detected at a certain node, this means that: (a) the substance was introduced into the network at that node, or (b) it originates from the upstream node. In this way, the presence of said unwanted chemical or biological substance propagates downstream following the network's causal structure. Therefore, the network takes on a Bayesian structure, where nodes are associated with their respective probabilities of encountering the undesirable chemical or biological substance. These probabilities are determined by the causal relationships between nodes and are provided by the same optimization algorithm that provides the set of nodes at which to perform the mobile sampling. Therefore, all nodes in the network are associated with a probability of being anomalous (the third purpose of the invention).

Finally, by repeating the monitoring on subsequent days, the temporal evolution of any anomaly can be verified (the fourth purpose of the invention).

**Detailed description of the invention**

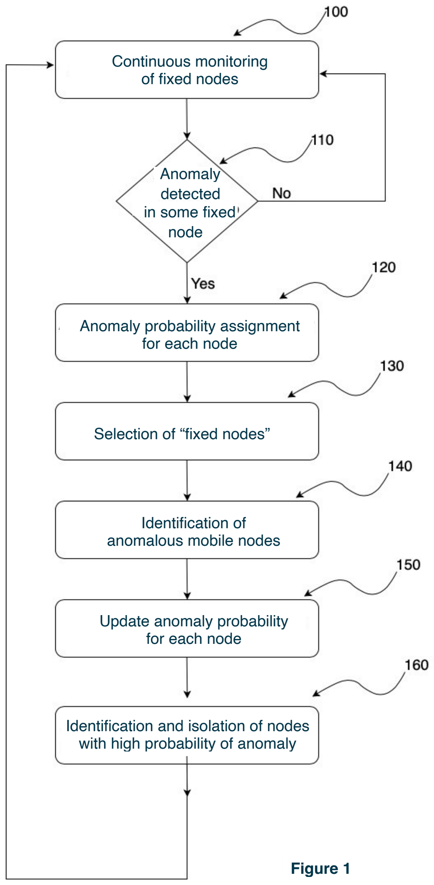

Figure 1 shows, by way of non-limiting example, a flowchart illustrating the main phases of the monitoring method proposed in the present invention.

The monitoring (100) of a predetermined set of network nodes, called "fixed nodes," is carried out continuously over time, which includes the following phases:

- definition of the chemical-physical-biological parameters to be monitored and of the anomalous value ranges for said parameters

- definition of a set of "fixed nodes" of the network at which to sample the materials flowing through the network

- scheduled analyses at regular time intervals, in situ or in a laboratory, of the samples taken from said "fixed nodes" and identification of any "anomalous fixed nodes," i.e., nodes for which some chemical-physical-biological parameter has an anomalous value.

In the event that the continuous monitoring of the fixed nodes detects any "anomalous fixed nodes" (110), a search is then carried out for all network nodes that could be sources of the anomaly, or involved in the infection even if not sources. This search includes the following phases:

a) Assignment of the probability $p$ for each network node to be anomalous (120)

For this purpose, consider the set of nodes denoted by $V$ and the set of edges E. An element $(i,j) \in$ E corresponds to a directed edge connecting node $i$ to node $j$. It is said that node $i$ is the "upstream" node, as water flows from $i$ to $j$, and $j$ is the "downstream" node.

When assigning probabilities, it is also possible to include physical parameters, such as dilution factors. For example, it is possible that the source node of the anomaly does not transmit it to its child nodes, precisely due to a dilution event. This can happen, for instance, in the transmission of a virus from one node to another: the viral load of a sample is dispersed and is no longer traceable.

Let $y_i$ be a Bernoulli random variable associated with a generic node $i \in V$, describing the presence ($y_i$=1) or absence ($y_i$=0) of an undesired chemical or biological substance at node $i$. In order to establish the presence or absence of an undesired chemical or biological substance, for example, a pollutant or a virus, chemical/physical/biological parameters are defined, for example, the concentration of a given substance, or the viscosity of the water, and the allowed value ranges for said parameters. Outside of these ranges, said parameters are considered anomalous and the probability $P(y_i)$ of the presence of said undesired chemical or biological substance is set to 1.

The anomaly of a node $i$ is determined by the sum of two contributions: from anomalies in the nodes upstream of $i$ and from the number of users connected to node $i$. Denoting with $\alpha_{ij}y_j$ the probability that a parent node $j$ transmits the anomaly to node $i$, and with $\alpha_{ij}$ a dilution factor that depends on the distance between $i$ and $j$, then the probability that the residues come from the population at node $i$ is encoded as $p_i^s = \gamma f\left(po\ p_i\right)$, where $f(pop_i)$ is a function of the population at node $i$ and $\gamma$ is a scaling factor to be set arbitrarily. Simple choices for $\alpha_{ij}$ and $f(pop_i)$ are $\alpha_{ij} = e^{\frac{-l_{ij}}{d}}$ and $f\left(po\ p_i\right) = 1 - e^{\frac{-pop_{ij}}{a}}$, where $l_{ij}$ is the physical distance between nodes $i$ and $j$, $d$ is a constant that depends on the characteristic length of the network, and a is a constant that depends on the average population. By factoring the contribution of the population and the Parent (Pa) nodes, the following expression for the probability of a generic node being anomalous is obtained (for fixed nodes, p = 1 if an anomaly has been detected, p = 0 otherwise):

$$p\left(y_i = 1 \middle| Pa_i\right) = 1 - \left(1 - p_i^s\right)\prod_{j \in Pa_i}\left(1 - a_{ij}\ y_j\right)$$

b) Selection of a new set of "mobile nodes" to be sampled, chosen from those not yet analyzed (130)

- define the number N of teams in the field and the number of samplings that each team can perform

- an uncertainty of being or not being anomalous is associated with each node. This uncertainty is defined as the Shannon entropy (conditioned on the analysis results of the fixed nodes s):

$$H_i = H\left(y_i\middle|s\right) = -\sum_{y_i=0,1} p\left(y_i\middle|s\right) log p\left(y_i\middle|s\right)$$

To this end, an energy function is defined, the minimum of which determines the set of said "mobile nodes." The energy function consists of five parts described below.

The sampling will be carried out by several teams in the field in successive steps. We, therefore, associate a binary variable $x_{i,t,a}$, with each node, team, and sampling time step. This variable will be equal to 1 if the corresponding node, team, and time step are selected by the algorithm for mobile sampling, and O otherwise. These variables, unlike the Bernoulli variables introduced above, are the variables on which the optimization is performed.

The first term of the energy function aims to choose the nodes with the highest Shannon entropy, calculated from the updated probabilities introduced above. Therefore, this term will correspond to the sum, over the set of nodes V, the set of teams $A=(1,...,A)$, and the time steps $T=(1,...,T)$, of the entropies per node multiplied by the corresponding binary variable. In the products of the summation, it is also appropriate to consider, for each node, a weight that refers to the fixed (static) sampling point upstream of the considered node: the aim is to prioritize and, therefore, to choose for mobile sampling those network nodes that are upstream of the fixed node where an anomaly was detected. We thus have for the first term of the energy function:

$$H_{(0)} = -\sum_{i \in V} \sum_{t \in T} \sum_{a \in A} c_i H_i x_{i,t,a}$$

It is very likely that spatially close nodes have similar entropy, and sampling both would be useless as no information would be gained. To this end, with the second term of the energy function, a repulsive term is included that tends to disadvantage the choice of statistically correlated nodes. The Mutual Information $I$ of each pair of network nodes is then calculated, again starting from the conditioned probabilities introduced above according to well-known formulas. The second term of the energy will consist of a double summation, over all pairs of nodes, teams, and time steps, of the mutual information of each pair of nodes multiplied by the product of the corresponding binary variables. We thus have for the second term of the energy function:

$$H_{(1)} = B \sum_{(i,j) \in V^2} \sum_{(t_1,t_2) \in T^2} \sum_{(a,b) \in A^2} I_{ij} x_{i,t_1,a} x_{j,t_2,b}$$

The third term is logistical in nature and analytically similar to the second. The goal is to choose a set of mobile nodes for which the teams in the field can perform the sampling within the established useful time, especially in relation to the physical movements the teams must make. To this end, in the third term of the energy function, a summation of the type just described for the second term is considered, where, however, the Mutual Information is replaced by the average travel time between each pair of nodes. This, in particular, can also be calculated from average traffic data provided by services like Google or OpenStreetMap. With this term, precisely, the aim is to disadvantage paths between pairs of nodes that are very distant from each other and that would require a total travel time greater than that established for the teams in the field to complete their task in a useful time. We thus have for the third term of the energy function:

$$H_{(2)} = C \sum_{(i,j) \in V^2} \sum_{t=0,...,T-1} \sum_{a \in A^2} T_{ij} x_{i,t,a} x_{j,t+1,a}$$

Obviously, the variables $x_{d,0,a} = x_{d,T,a} = 1$ are fixed at 1 for each team, since all teams start from and return to the same point $d$ (depot).

With the fourth and fifth terms of the energy function, the goal is to introduce constraints on the type of optimization. In particular, it is important that at each step of the sampling and for each team, the designated node for sampling is unique. For this, for each team in the field and each time interval, the summation over the nodes of all binary spin variables must be equal to one. To this end, following QUBO logic, a quadratic penalty term is inserted as just described:

$$H_{(3)} = D \sum_{t \in T} \sum_{a \in A} \left(\sum_{i \in V} x_{i,t,a} - 1\right)^2$$

With the fifth term, however, the aim is to ensure that the binary spin variable associated with each network node, time interval, and team is always equal to or less than one. In particular, it is appropriate that the same node is not chosen twice by the optimizer, for example, for sampling in two different time steps or by two different teams. Since this is an inequality constraint, it is necessary to consider slack variables, as many as there are network nodes. The implementation, moreover, is always done by considering a quadratic term of the energy:

$$H_{(4)} = E \sum_{i \in V} \left(\sum_{t \in T} \sum_{a \in A} x_{i,t,a} + y_i - 1\right)^2$$

Finally, it is important to note that if, during sampling operations, one of the points identified by the algorithm should be inaccessible due to an unforeseen event, it is possible to run the algorithm again excluding the point in question. Since the execution times of the algorithm are very fast, it is possible to perform everything in real-time. In this way, unforeseen events related to mobile sampling can be easily overcome, and the campaign can be completed in a useful time.

(c) analysis, in situ or in a laboratory, of the samples taken from said "mobile nodes" and identification of any anomalous "mobile nodes" (140).

(d) updating of the probabilities $p$ for each network node to be anomalous (150)

(e) said probabilities $p$ for each network node to be anomalous are updated, and the nodes with a high probability $p$ of being anomalous, i.e., involved in an infection, are identified for a more detailed check until the anomaly is resolved. For example, the nodes with the highest probability of being anomalous are isolated for more granular checks until the anomaly is resolved (160).

**Claims**

1. Method of monitoring pressure and/or gravity networks, for example sewer networks, comprising the phases of:

   - continuous monitoring of a set of nodes, called "fixed nodes", of the network (100)
   - in the event of detection of anomalous "fixed nodes", search for all nodes of the network that could be sources of the detected anomaly

   **characterised in that**

   1) said search for all nodes of the network that could be sources of the detected anomaly comprises the following consecutive steps:

   a) assignment to each node of the network of a probability p of being anomalous, on the basis of the structure of the network and conditioned on the results of the analyses of said "fixed nodes" (120)
   b) selection of a set of nodes to be sampled, called "mobile nodes", chosen from those not yet analysed (130)
   c) analysis of the samples taken in said "mobile nodes" and identification of anomalous "mobile nodes" (140)
   d) on the basis of the results of the analyses worked in the previous phase, update the probabilities p associated with each node of the network of being anomalous (150)
   e) identification and isolation of the nodes with high probability p of being sources of the anomaly (160)

2. method as from claim 1, wherein the selection of said "mobile nodes" is carried out on the basis of both said probabilities p of each node of being anomalous, and on the basis of the logistical constraints by applying a probability calculation algorithm in which said probabilities p associated with each node of the network of being anomalous are conditioned by the logistical constraints

3. method as from claim 2, wherein said logistical constraints include:

   - accessibility to the nodes
   - travel times to reach the nodes
   - availability of human resources and material means to reach the nodes

4. method as from claim 2, wherein:

   - said probability calculation algorithm is coded in a suitable way for being executed on a quantum computer

**Figure 1**

**EP 4 726 125 A1**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 112 960 A1 (SUEZ GROUPE [FR]) 4 January 2017 (2017-01-04) | 1-3 | INV. E03F7/00 |
| Y | * paragraph [0011] - paragraph [0141] * ----- | 4 | F17D5/02 G01M3/28 |
| X | SPEZIALI STEFANO ET AL: "A Novel Approach to Face Early Pandemics Using QUBO Models", 2023 IEEE INTERNATIONAL CONFERENCE ON QUANTUM COMPUTING AND ENGINEERING (QCE), IEEE, vol. 2, 17 September 2023 (2023-09-17), pages 266-267, XP034478878, DOI: 10.1109/QCE57702.2023.10236 * the whole document * ----- | 1 | G01N33/18 G05B13/04 G06F30/27 G06N20/00 G06F21/57 |
| Y | US 2023/252335 A1 (SEWELL JONATHAN [US] ET AL) 10 August 2023 (2023-08-10) * paragraph [0019] - paragraph [0070] * ----- | 4 | |

**TECHNICAL FIELDS SEARCHED (IPC)**

E03F
G06F
G01M
G05B
G01N
G06N
F17D

The present search report has been drawn up for all claims

| Place of search | Date of completion of the search | Examiner |
|---|---|---|
| Munich | 28 January 2026 | Veshi, Erzim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 7400

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2026

| Patent document cited in search report | | Publication date | Patent family member(s) | | Publication date |
|---|---|---|---|---|---|
| EP 3112960 | A1 | 04-01-2017 | AU | 2016286280 A1 | 18-01-2018 |
| | | | AU | 2016287383 A1 | 18-01-2018 |
| | | | BR | 112017028077 A2 | 28-08-2018 |
| | | | CL | 2017003377 A1 | 15-06-2018 |
| | | | CL | 2017003382 A1 | 15-06-2018 |
| | | | CN | 107949812 A | 20-04-2018 |
| | | | CN | 108027594 A | 11-05-2018 |
| | | | EP | 3112959 A1 | 04-01-2017 |
| | | | EP | 3112960 A1 | 04-01-2017 |
| | | | ES | 2784233 T3 | 23-09-2020 |
| | | | ES | 2906411 T3 | 18-04-2022 |
| | | | MX | 390105 B | 20-03-2025 |
| | | | MX | 394253 B | 24-03-2025 |
| | | | SG | 10201910834T A | 30-01-2020 |
| | | | US | 2018181111 A1 | 28-06-2018 |
| | | | US | 2019004484 A1 | 03-01-2019 |
| | | | WO | 2017001522 A1 | 05-01-2017 |
| | | | WO | 2017001523 A1 | 05-01-2017 |
| US 2023252335 | A1 | 10-08-2023 | NONE | | |

For more details about this annex : see Official Journal of the European Patent Office, No. 12/82